# EUROPÄISCHE PATENTSCHRIFT

(11) **EP 3 426 175 B1**
(45) Veröffentlichungstag und Bekanntmachung des Hinweises auf die Patenterteilung: **19.06.2024**
(21) Anmeldenummer: 17709071.9
(22) Anmeldetag: 07.03.2017
(51) Int. Cl.: A61B 18/14, A61B 1/00

(54) **RESEKTOSKOP UND ELEKTRODENANORDNUNG DAFÜR**
RESECTOSCOPE AMD ELECTRODE ASSEMBLY THEREFOR
RÉSECTOSCOPE ET ENSEMBLE D'ÉLECTRODES PRÉVU À CET EFFET

(30) Priorität: 11.03.2016 DE 102016204047
(43) Veröffentlichungstag der Anmeldung: 16.01.2019
(73) Patentinhaber: Olympus Winter & Ibe GmbH, 22045 Hamburg (DE)
(72) Erfinder: STÜHLE, Sebastian, 45721 Haltern am See (DE); KAPFERMANN, Nils, 22043 Hamburg (DE); FREITAG, Thomas, 22089 Hamburg (DE); KAISER, Andreas, 22089 Hamburg (DE); BROCKMANN, Christian, 21279 Hollenstedt (DE)
(74) Vertreter: Glawe, Delfs, Moll
(86) Internationale Anmeldenummer: PCT/EP2017/055263
(87) Internationale Veröffentlichungsnummer: WO 2017/153374

(56) Entgegenhaltungen:
- DE-A1- 2 528 543
- DE-A1-102014 212 102
- US-A1- 2011 295 066
- US-A1- 2014 039 486

## Beschreibung

Die Erfindung betrifft ein Resektoskop sowie eine Elektrodenanordnung dafür.

Resektoskope werden bei minimal-invasiven chirurgischen Eingriffen vor allem in der Urologie, aber beispielsweise auch in der Gynäkologie verwendet. Sie weisen an ihrem distalen Ende eine oder mehrere freiliegende Elektroden zur Gewebebearbeitung auf. Bei den Elektroden kann es sich um monopolare oder bipolare Elektroden handeln. Es ist aber auch möglich, dass die Elektrode als freies Ende einer Laserfaser ausgebildet ist.

Resektoskope weisen in der Regel ein langgestrecktes Schaftrohr zur Einführung in den Körper des Patienten auf. In diesem Schaftrohr ist eine ebenfalls langgestreckte Optik angeordnet, an deren distalem Ende ein Objektiv vorgesehen ist. Am anderen Ende der Optik kann ein Okular vorgesehen sein, durch welches der Operateur den Operationsbereich vor dem Objektiv einsehen kann.

Ebenfalls im Schaftrohr angeordnet ist eine längsverschiebbare Elektrodenanordnung mit einer Elektrode am distalen Ende. Die Elektrodenanordnung lässt sich zwischen einem eingefahrenen Zustand, bei dem die Elektrode innerhalb des Schaftrohrs und unmittelbar vor dem Objektiv angeordnet ist, und einem ausgefahrenen Zustand, bei der die Elektrode freiliegend aus dem Schaftrohr ragt, verschieben. Ein solches Resektoskop ist aus dem Dokument US 2011/0295066 A1 bekannt.

Im ausgefahrenen Zustand befindet sich die Elektrode im Blickfeld der Optik. Um eine gute Sicht auf die Elektrode zu haben, wird der Operationsbereich derart mit einer Spülflüssigkeit durchgespült, dass sich ein konstanter Druck im Operationsbereich ergibt. Gleichzeitig soll durch geeignete Anordnung aus Spülflüssigkeitszufuhr und Absaugung gewährleistet werden, dass der Bereich unmittelbar vor der Optik möglichst frei von die Sicht behindernden Verschmutzungen - bspw. aufgrund von ausgetretenem Blut oder freischwebenden Gewebeteilen - bleibt. Ein maßgebliches Kriterium hierfür ist die Zeit, die benötigt wird, bis das Blickfeld der Optik nach einer erfolgten Verschmutzung wieder frei ist.

Um eine zur Freispülen des Blickfeldes geeignete Umströmung des Objektivs zu erreichen, erfolgt zumindest die Spülflüssigkeitszufuhr regelmäßig direkt über das Resektoskop. Dennoch ist die Zeit, die benötigt wird, das Blickfeld vor der Optik nach einer Verschmutzung wieder frei zu spülen, häufig unbefriedigend lang.

Der Erfindung liegt die Aufgabe zugrunde, ein Resektoskop sowie eine Elektrodenanordnung hierfür zu schaffen, bei der die Nachteile aus dem Stand der Technik nicht mehr oder nur noch im verminderten Umfang auftreten.

Gelöst wird diese Aufgabe durch ein Resektoskop gemäß dem Hauptanspruch sowie eine Elektrodenanordnung gemäß dem nebengeordneten Anspruch 10. Vorteilhafte Weiterbildungen sind Gegenstand der abhängigen Ansprüche.

Demnach betrifft die Erfindung ein Resektoskop umfassend ein langgestrecktes Schaftrohr, eine darin angeordnete langgestreckte Optik mit einem Objektiv am distalen Ende und einer Elektrodenanordnung, wobei die Elektrodenanordnung eine Elektrode umfasst, die am distalen Ende der Arme einer Gabelbaugruppe angeordnet ist, die auf gegenüberliegenden Seiten des Objektivs angeordnet und in einem Übergangsbereich zu einem Elektrodenschaft zusammengeführt sind, wobei die Elektrodenanordnung in Längsrichtung um eine Hublänge von einer ersten Position, bei der die Elektrode innerhalb des Schaftrohrs vor dem Objektiv angeordnet ist, in eine zweite Position, in der die Arme der Gabelbaugruppe aus dem Schaftrohr ragen, ausfahrbar ist, wobei die Hublänge die Länge zwischen den Endpunkten der Bewegbarkeit der Elektrodenanordnung bezeichnet und wobei die Länge der Arme der Gabelbaugruppe größer ist als das 1,2-fache der Hublänge.

Die Erfindung betrifft außerdem eine Elektrodenanordnung zur Verwendung in einem (vorzugsweise erfindungsgemäßen) Resektoskop mit einer Hublänge von 24 mm, bei der die Elektrodenanordnung um die Hublänge in Längsrichtung ausfahrbar ist, umfassend eine Elektrode, die am distalen Ende der Arme einer Gabelbaugruppe angeordnet ist, die in einem Übergangsbereich zu einem Elektrodenschaft zusammengeführt sind, wobei die Länge der Arme der Gabelbaugruppe größer ist als das 1,2-fache der Hublänge.

Der Erfindung liegt die Erkenntnis zugrunde, dass zumindest bei aus dem Stand der Technik bekannten Elektrodenanordnungen, die - vergleichbar zu derjenigen der Erfindung - eine Gabelbaugruppe, einen Übergangsbereich und einen Elektrodenschaft umfassen, bei denen die Länge der Arme der Gabelbaugruppe jedoch im Wesentlichen der Hublänge entspricht, die Übergangsbereiche regelmäßig Verwirbelungen in der strömenden Spülflüssigkeit im Bereich unmittelbar vor dem Objektiv verursachen, welche dem Freispülen des Blickfeldes abträglich sind. Bei einer entsprechenden Länge der Arme der Gabelbaugruppe befindet sich der Übergangsbereich im ausgefahrenen Zustand nämlich unmittelbar benachbart zum Objektiv und kann dann die unerwünschten Verwirbelungen in der strömenden Spülflüssigkeit im Bereich unmittelbar vor dem Objektiv verursachen.

Indem erfindungsgemäß vorgesehen ist, die Länge der Arme der Gabelbaugruppe deutlich - nämlich um wenigstens 20% - größer als die Hublänge zu wählen, wird erreicht, dass der Übergangsbereich der Elektrodenanordnung auch im ausgefahrenen Zustand derart ausreichend beabstandet von dem Objektiv der Optik angeordnet ist, dass durch den Übergangsbereich keine oder gegenüber dem Stand der Technik weniger, das Freispülen des Blickfeldes verzögernde Verwirbelungen in der Spülflüssigkeit entstehen.

Es ist bevorzugt, wenn die Länge der Arme der Gabelbaugruppe größer ist als das 1,3-fache der Hublänge, vorzugsweise größer ist als das 1,4-fache der Hublänge, weiter vorzugsweise größer ist als das 1,5-fache der Hublänge. Durch eine entsprechende Länge der der Arme der Gabelbaugruppe kann der störende Einfluss des Übergangsbereichs auf die Strömung im Bereich von dem Objektiv weiter verringert werden. Um eine ausreichende Stabilität der Gabelbaugruppe zu gewährleisten, ist es bevorzugt, wenn die die Länge der Arme der Gabelbaugruppe das 1,8-fache der Hublänge nicht überschreitet.

Der Übergangsbereich der beiden Arme der Gabelbaugruppe zum Elektrodenschaft kann jeweils als S-Schlag ausgebildet sein. Bei einem S-Schlag handelt es sich um einen fließend geschwungenen Übergang, der an eine S-Form erinnert und im Wesentlichen ohne abrupte Winkelveränderung tangential von einem Arm der Gabelbaugruppe tangential zum Elektrodenschaft führt. Zur besseren Führung der Elektrodenanordnung im Bereich des distalen Endes des Schaftrohrs kann am Elektrodenschaft benachbart zum Übergangsbereich ein Führungselement vorgesehen sein, welches mit der Optik derart zusammenwirkt, dass der Elektrodenschaft entlang der Optik geführt wird. Das Führungselement kann bspw. rohrförmig ausgebildet sein, um insbesondere im Falle eines kreisrundförmigen Querschnitts der Optik diese ganz oder zumindest teilweise zu umgreifen. Bei einem teilweisen Umgreifen erstreckt sich das Führungselement über wenigstens 60% des Umfangs der Optik.

Die Vorteile der Erfindung lassen sich bei verschiedenen Bauarten von Resektoskopen und Arten der Spülflüssigkeitszuführung erreichen. Insbesondere kommen die Vorteile der Erfindung aber zum Tragen, wenn der Bereich zwischen dem Schaftrohr und der Optik als Spülflüssigkeitszuführung ausgebildet ist. Die Spülflüssigkeit strömt dabei an der Optik entlang durch den Bereich, in dem sich auch die Elektrodenanordnung befindet, und tritt am distalen Ende des Resektoskops aus. Strömungsbeeinflussungen in dem Spülflüssigkeitsstrom, die insbesondere aufgrund des Übergangsbereichs der Elektrodenanordnungen entstehen können, sind aufgrund der erfindungsgemäßen Ausgestaltung der Elektrodenanordnung auch im ausgefahrenen Zustand ausreichend weit von dem Objektiv der Optik entfernt angeordnet, dass sich aufgrund dieser Strömungsbeeinflussungen keine oder nur wenige Verwirbelungen im Bereich unmittelbar vor dem Objektiv ergeben.

Das Schaftrohr ist vorzugsweise doppelwandig ausgebildet, wobei der Bereich zwischen den Wänden des Schaftrohrs als Spülmittelabfluss ausgebildet ist. Die Spülflüssigkeit kann bei einer entsprechenden Ausgestaltung durch das Resektoskop sowohl zu- als auch abgeführt werden, sodass bei einem Eingriff keine gesonderten Spülflüssigkeitszu- oder -abführungsvorrichtungen vorgesehen werden müssen.

Die Elektrode der Elektrodenanordnung ist vorzugsweise eine bipolare Elektrode. Sie ist weiter vorzugsweise als Schlingenelektrode oder als flächige Vaporisationselektrode ausgebildet.

Die Hublänge des Resektoskops beträgt vorzugsweise 24 mm. Die Länge der Arme der Gabelbaugruppe beträgt dann vorzugsweise 30 mm bis 28 mm, weiter vorzugsweise 32 mm bis 34 mm.

Zur Erläuterung der erfindungsgemäßen Elektrodenanordnung wird auf die vorstehenden Ausführungen verwiesen.

Die Erfindung wird nun anhand vorteilhafter Ausführungsformen unter Bezugnahme auf die beigefügten Zeichnungen beispielhaft beschrieben. Es zeigen:
- Figur 1:: ein Ausführungsbeispiel eines erfindungsgemäßen Resektoskops mit eingefahrener Elektrodenanordnung;
- Figur 2:: das Resektoskop aus Figur 1 mit ausgefahrener Elektrodenanordnung;
- Figur 3:: ein erstes Ausführungsbeispiel einer erfindungsgemäßen Elektrodenanordnung; und
- Figur 4:: ein zweites Ausführungsbeispiel einer erfindungsgemäßen Elektrodenanordnung.

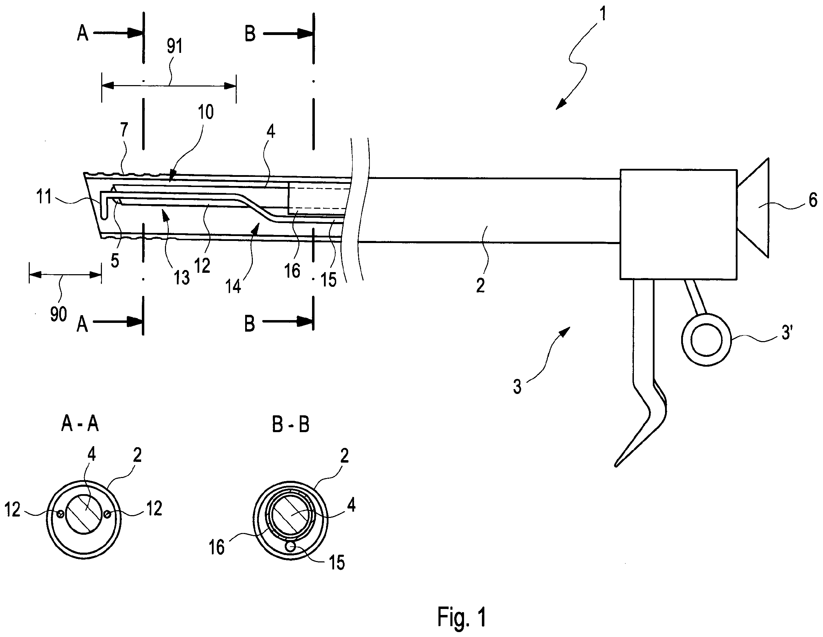

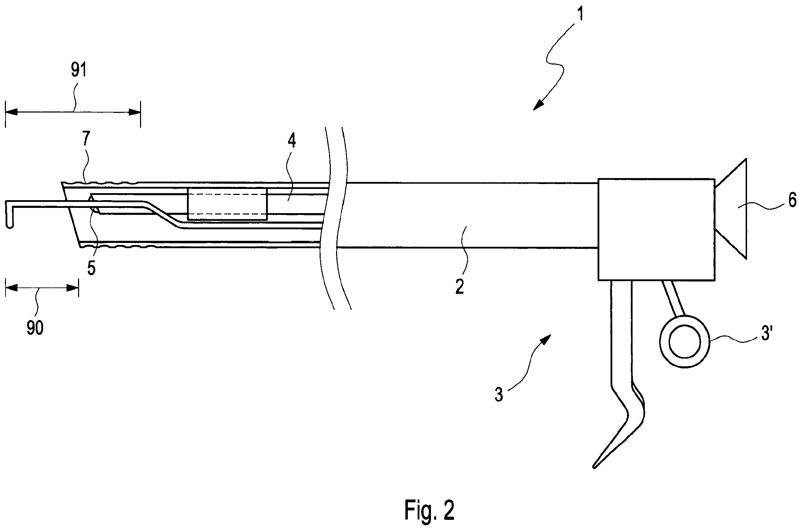

In Figuren 1 und 2 ist ein erfindungsgemäßes Resektoskop 1 dargestellt. Das Resektoskop 1 umfasst ein langgestrecktes Schaftrohr 2, dessen distales Ende zum Einführen in den Körper eines Patienten ausgebildet ist. An seinem anderen Ende ist das Schaftrohr mit einer Griffanordnung 3 verbunden.

In dem Schaftrohr 2 ist eine ebenfalls langgestreckte Optik 4 angeordnet, die am distalen Ende ein Objektiv 5 aufweist. Am entgegengesetzten Ende der Optik 4 ist ein an der Griffanordnung 3 angeordnetes Okular 6 vorgesehen.

Der Bereich zwischen der Optik 4 und dem Schaftrohr 2 ist als Spülflüssigkeitszuführung ausgebildet. Dabei wird über einen (nicht dargestellten) Anschluss an der Griffanordnung 3 Spülflüssigkeit zugeführt, die durch den Zwischenraum zwischen Schaftrohr 2 und Optik 4 strömt und am distalen Ende des Schaftrohrs 2 austritt.

Das Schaftrohr 4 ist doppelwandig ausgestaltet und weist am distalen Ende an der Außenseite Einlassöffnungen 7 auf. Durch diese Einlassöffnungen 7 kann Spülflüssigkeit angesaugt werden, die dann zwischen den beiden Wänden des Schaftrohrs 2 zur Griffanordnung 3 und dort über einen (nicht dargestellten) Anschluss abgeleitet wird.

Da sowohl die Spülflüssigkeitszufuhr als auch die Spülflüssigkeitsabführung unmittelbar durch das Resektoskop 1 erfolgt, müssen für einen Eingriff keine gesonderten Einrichtungen für die Zu- oder Abführung von Spülflüssigkeit vorgesehen werden.

Das Resektoskop 1 umfasst weiterhin eine Elektrodenanordnung 10, die über ein Griffelement 3` der Griffanordnung 3 zwischen dem in Figur 1 dargestellten eingefahrenen Zustand zu dem in Figur 2 dargestellten ausgefahrenen Zustand bewegt werden kann. Die Länge, um die sich die Elektrodenanordnung 10 zwischen den in Figuren 1 und 2 dargestellten Endpunkten bewegt, ist als Hublänge 90 bezeichnet und beträgt im dargestellten Ausführungsbeispiel 24 mm.

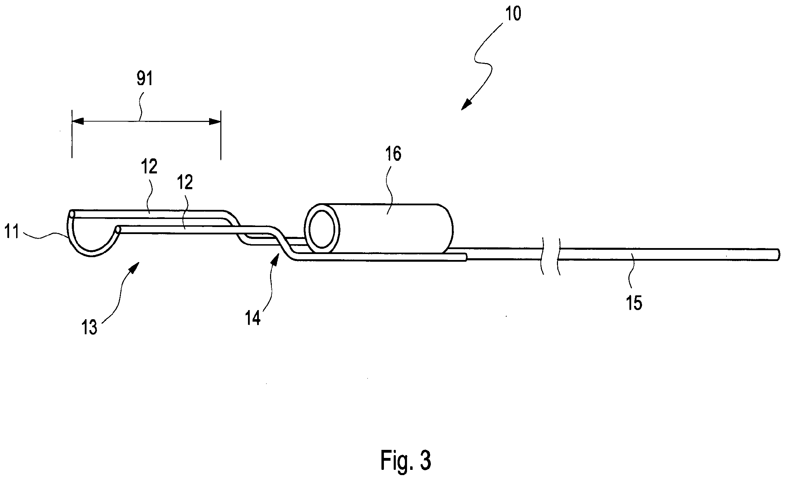

Die im Resektoskop 1 gemäß Figur 1 eingesetzte Elektrodenanordnung 10 - die derjenigen aus in Figur 3 entspricht - umfasst an ihrem distalen Ende eine bipolare Schlingenelektrode 11. Die Schlingenelektrode 11 ist dabei an den äußeren Enden zweier Arme 12 einer Gabelbaugruppe 13 angeordnet. Die Arme 12 der Gabelbaugruppe 13 sind an gegenüberliegenden Seiten der Optik 4 und somit auch des Objektivs 5 angeordnet (vgl. Figur 1, Schnitt A-A). Über einen als S-Schlag ausgebildeten Übergangsbereich 14 werden die beiden Arme 12 der Gabelbaugruppe 13 zu einem Elektrodenschaft 15 zusammengeführt. Der Elektrodenschaft 15 erstreckt sich bis in die Griffanordnung 3 und ist dort zum einem für die beschriebene Längsverschiebbarkeit mit dem Griffelement 3', zum anderen mit (nicht dargestellten) Anschlüssen für die Zuführung von hochfrequenter elektrischer Energie verbunden. Diese Anbindungen sind gemäß dem Stand der Technik ausgebildet.

Zu Führung der Elektrodenanordnung 10 im von der Griffanordnung 3 entfernten Bereich ist ein benachbart zum Übergangsbereich 14 mit dem Elektrodenschaft 15 verbundenes rohrförmiges Führungselement 16 vorgesehen, welches die Optik 4 vollständig umgreift und entlang dieser gleiten kann.

Wie in Figuren 1 und 2 und insbesondere in den Schnitten A-A und B-B der Figur 1 ersichtlich, ist die Elektrodenanordnung 10 in dem Bereich zwischen Schaftrohr 2 und Optik 4 angeordnet, der auch zur Zuführung von Spülflüssigkeit genutzt wird. Insbesondere durch den Übergangsbereich 14 der Elektrodenanordnung 10 kommt es zu Verwirbelungen in der Spülflüssigkeitsströmung, die für ein Freispülen des Blickfeldes der Optik 4 bzw. des Objektivs 5 nachteilig sein können. Bei dem in Figur 1 und 2 dargestellten Resektoskop 1 sind die Arme der 12 der Gabelbaugruppe 13 jedoch um mehr als das 1,2-fache der Hublänge 90 länger, sodass sich - wie in Figur 2 dargestellt - der Übergangsbereich 14 auch im ausgefahrenen Zustand der Elektrodenanordnung 10 ausreichend weit vom Objektiv 5 entfernt befindet, dass sich durch den Übergangsbereich 14 im Spulflüssigkeitszustrom ergebene Verwirbelungen im Bereich des Objektivs 5 wieder ausreichend beruhigt haben, sodass das Blickfeld der Optik 4 bzw. des Objektivs 5 bei Verschmutzung zügig freigespült wird. Die Länge 91 der Arme 12 der Gabelbaugruppe 13 im dargestellten Ausführungsbeispiel beträgt 35 mm.

Das in Figur 3 dargestellte Ausführungsbeispiel einer erfindungsgemäßen Elektrodenanordnung 10 wurde bereits in Zusammenhang mit dem Resektoskop 1 aus Figuren 1 und 2 erläutert, weshalb auf die entsprechenden Ausführungen verwiesen wird.

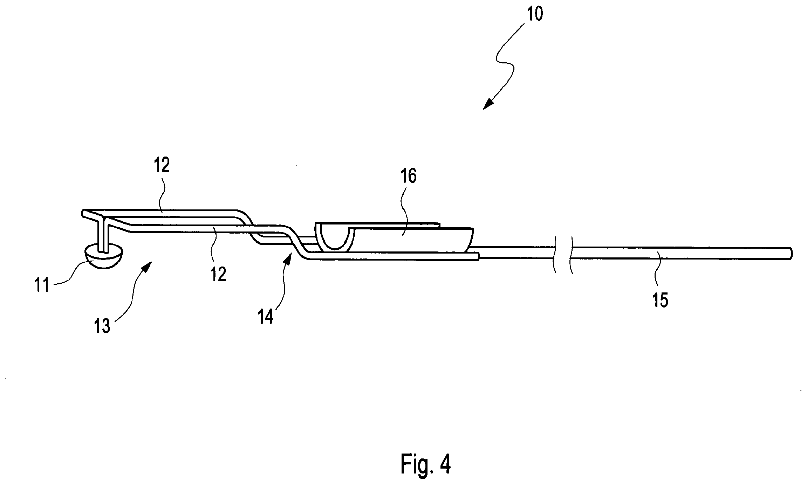

In Figur 4 ist ein alternatives Ausführungsbeispiel einer erfindungsgemäßen Elektrodenanordnung 10. Der grundsätzliche Aufbau der Elektrodenanordnung 10 mit Gabelbaugruppe 13, einem Übergangsbereich 14 und einem Elektrodenschaft 15 entspricht dabei demjenigen des Ausführungsbeispiels aus Figur 3. Im Unterschied dazu ist bei dem Ausführungsbeispiel gemäß Figur 4 die Elektrode 11 jedoch als flächige Vaporisationselektrode ausgebildet. Außerdem ist das Führungselement 16 kein geschlossen-rohrförmiges Element, sondern weist einen kreisringsegmentförmigen Querschnitt auf. Das Kreisringsegment erstreckt sich dabei um 220°, sodass bspw. bei dem Resektoskop 1 gemäß Figuren 1 und 2 die Optik von dem Führungselement 16 um mehr als 60% ihres Umfangs umgriffen würde, womit eine ausreichende Führung der Elektrodenanordnung 10 an der Optik 4 hergestellt werden kann.

## Patentansprüche

1. Resektoskop (1) umfassend ein langgestrecktes Schaftrohr (2), eine darin angeordnete langgestreckte Optik (4) mit einem Objektiv (5) am distalen Ende und einer Elektrodenanordnung (10), wobei die Elektrodenanordnung (10) eine Elektrode (11) umfasst, die am distalen Ende der Arme (12) einer Gabelbaugruppe (13) angeordnet ist, die auf gegenüberliegenden Seiten des Objektivs (5) angeordnet und in einem Übergangsbereich (14) zu einem Elektrodenschaft (15) zusammengeführt sind, wobei die Elektrodenanordnung (10) in Längsrichtung um eine Hublänge (90) von einer ersten Position, bei der die Elektrode (11) innerhalb des Schaftrohrs (2) vor dem Objektiv (5) angeordnet ist, in eine zweite Position, in der die Arme (12) der Gabelbaugruppe (13) aus dem Schaftrohr (2) ragen, ausfahrbar ist, wobei die Hublänge (90) die Länge zwischen den Endpunkten der Bewegbarkeit der Elektrodenanordnung (10) bezeichnet und wobei die Länge (91) der Arme (12) der Gabelbaugruppe (13) größer ist als das 1,2-fache der Hublänge (90).

2. Resektoskop nach Anspruch 1,
**dadurch gekennzeichnet, dass**
die Länge (91) der Arme (12) der Gabelbaugruppe (13) größer ist als das 1,3-fache der Hublänge (90), vorzugsweise größer ist als das 1,4-fache der Hublänge (90), weiter vorzugsweise größer ist als das 1,5-fache der Hublänge (90) ist.

3. Resektoskop nach einem der vorhergehenden Ansprüche,
**dadurch gekennzeichnet, dass**
der Übergangsbereich (14) der beiden Arme (12) der Gabelbaugruppe (13) zum Elektrodenschaft (15) jeweils als S-Schlag ausgebildet ist.

4. Resektoskop nach einem der vorhergehenden Ansprüche,
**dadurch gekennzeichnet, dass**
am Elektrodenschaft (15) benachbart zum Übergangsbereich (14) ein Führungselement (16) vorgesehen ist, welches mit der Optik (4) derart zusammenwirkt, dass der Elektrodenschaft (15) entlang der Optik (4) geführt wird.

5. Resektoskop nach dem vorhergehenden Anspruch,
**dadurch gekennzeichnet, dass**
das Führungselement (16) rohrförmig ist.

6. Resektoskop nach einem der vorhergehenden Ansprüche,
**dadurch gekennzeichnet, dass**
der Bereich zwischen dem Schaftrohr (2) und der Optik (4) als Spülflüssigkeitszuführung ausgebildet ist.

7. Resektoskop nach einem der vorhergehenden Ansprüche,
**dadurch gekennzeichnet, dass**
das Schaftrohr (2) doppelwandig ist, wobei der Bereich zwischen den Wänden des Schaftrohrs (2) als Spülmittelabfluss ausgebildet ist.

8. Resektoskop nach einem der vorhergehenden Ansprüche,
**dadurch gekennzeichnet, dass**
die Elektrode (11) der Elektrodenanordnung (10) eine bipolare Elektrode, vorzugsweise eine Schlingenelektrode oder eine flächige Vaporisationselektrode ist.

9. Resektoskop nach einem der vorhergehenden Ansprüche,
**dadurch gekennzeichnet, dass**
die Hublänge (90) 24 mm ist und/oder die Länge (91) der Arme (12) der Gabelbaugruppe (13) 30 mm bis 28 mm, vorzugsweise 32 mm bis 34 mm ist.

10. Elektrodenanordnung (10) zur Verwendung in einem Resektoskop (1) mit einer Hublänge von 24 mm, bei der die Elektrodenanordnung (10) um die Hublänge (90) in Längsrichtung ausfahrbar ist, wobei die Hublänge (90) die Länge zwischen den Endpunkten der Bewegbarkeit der Elektrodenanordnung (10) bezeichnet, umfassend eine Elektrode (11), die am distalen Ende der Arme (12) einer Gabelbaugruppe (13) angeordnet ist, die in einem Übergangsbereich (14) zu einem Elektrodenschaft (15)zusammengeführt sind, wobei die Länge (91) der Arme (12) der Gabelbaugruppe (13) größer ist als das 1,2-fache der Hublänge (90).

## Claims

1. Resectoscope (1) comprising an elongate shaft tube (2), an elongate optic (4) arranged therein with an objective lens (5) at the distal end and with an electrode assembly (10), wherein the electrode assembly (10) comprises an electrode (11) arranged at the distal end of the arms (12) of a fork assembly (13), which are arranged on opposite sides of the objective lens (5) and are brought together in a transition region (14) to an electrode shaft (15), wherein the electrode assembly (10) can be deployed in the longitudinal direction by a stroke length (90) from a first position, in which the electrode (11) is arranged inside the shaft tube (2) in front of the objective lens (5), to a second position, in which the arms (12) of the fork assembly (13) protrude from the shaft tube (2), wherein the stroke length (90) designates the length between the end points of the mobility of the electrode assembly (10), and wherein the length (91) of the arms (12) of the fork assembly (13) is greater than 1.2 times the stroke length (90).

2. Resectoscope according to Claim 1, **characterized in that** the length (91) of the arms (12) of the fork assembly (13) is greater than 1.3 times the stroke length (90), preferably greater than 1.4 times the stroke length (90), more preferably greater than 1.5 times the stroke length (90) .

3. Resectoscope according to one of the preceding claims, **characterized in that** the transition region (14) of the two arms (12) of the fork assembly (13) to the electrode shaft (15) is designed in each case as a reflexed airfoil profile.

4. Resectoscope according to one of the preceding claims, **characterized in that** a guide element (16) is provided on the electrode shaft (15), adjacent to the transition region (14), and interacts with the optic (4) in such a way that that the electrode shaft (15) is guided along the optic (4).

5. Resectoscope according to the preceding claim, **characterized in that** the guide element (16) is tubular.

6. Resectoscope according to one of the preceding claims, **characterized in that** the region between the shaft tube (2) and the optic (4) is configured for rinsing liquid delivery.

7. Resectoscope according to one of the preceding claims, **characterized in that** the shaft tube (2) is doublewalled, wherein the region between the walls of the shaft tube (2) is configured for rinsing medium discharge.

8. Resectoscope according to one of the preceding claims, **characterized in that** the electrode (11) of the electrode assembly (10) is a bipolar electrode, preferably a loop electrode or a flat vaporization electrode.

9. Resectoscope according to one of the preceding claims, **characterized in that** the stroke length (90) is 24 mm and/or the length (91) of the arms (12) of the fork assembly (13) is 30 mm to 28 mm, preferably 32 mm to 34 mm.

10. Electrode assembly (10) for use in a resectoscope (1), with a stroke length of 24 mm, in which the electrode assembly (10) can be deployed in the longitudinal direction by the stroke length (90), wherein the stroke length (90) designates the length between the end points of the mobility of the electrode assembly (10), comprising an electrode (11) arranged at the distal end of the arms (12) of a fork assembly (13), which are brought together in a transition region (14) to an electrode shaft (15), wherein the length (91) of the arms (12) of the fork assembly (13) is greater than 1.2 times the stroke length (90) .

## Revendications

1. Résectoscope (1) comprenant un tube de tige allongé (2), une optique allongée (4) agencée dans celui-ci avec un objectif (5) à l'extrémité distale et un agencement d'électrode (10), l'agencement d'électrode (10) comprenant une électrode (11) qui est agencée à l'extrémité distale des bras (12) d'un sous-groupe de fourche (13) qui sont agencés sur des côtés opposés de l'objectif (5) et qui sont réunis dans une zone de transition (14) en une tige d'électrode (15), l'agencement d'électrode (10) pouvant être déployé dans la direction longitudinale d'une longueur de course (90) d'une première position, dans laquelle l'électrode (11) est agencée à l'intérieur du tube de tige (2) devant l'objectif (5), à une deuxième position, dans laquelle les bras (12) du sous-groupe de fourche (13) font saillie du tube de tige (2), la longueur de course (90) désignant la longueur entre les points d'extrémité de la mobilité de l'agencement d'électrode (10), et la longueur (91) des bras (12) du sous-groupe de fourche (13) étant supérieure à 1,2 fois la longueur de course (90).

2. Résectoscope selon la revendication 1,
**caractérisé en ce que**
la longueur (91) des bras (12) du sous-groupe de fourche (13) est supérieure à 1,3 fois la longueur de course (90), de préférence est supérieure à 1,4 fois la longueur de course (90), de manière davantage préférée est supérieure à 1,5 fois la longueur de course (90).

3. Résectoscope selon l'une quelconque des revendications précédentes,
**caractérisé en ce que**
la zone de transition (14) des deux bras (12) du sous-groupe de fourche (13) vers la tige d'électrode (15) est configurée respectivement sous la forme d'une jonction en S.

4. Résectoscope selon l'une quelconque des revendications précédentes,
**caractérisé en ce que**
sur la tige d'électrode (15), au voisinage de la zone de transition (14), il est prévu un élément de guidage (16) qui coopère avec l'optique (4) de telle sorte que la tige d'électrode (15) est guidée le long de l'optique (4).

5. Résectoscope selon la revendication précédente,
**caractérisé en ce que**
l'élément de guidage (16) est tubulaire.

6. Résectoscope selon l'une quelconque des revendications précédentes,
**caractérisé en ce que**
la zone entre le tube de tige (2) et l'optique (4) est configurée sous forme d'amenée de liquide de rinçage.

7. Résectoscope selon l'une quelconque des revendications précédentes,
**caractérisé en ce que**
le tube de tige (2) est à double paroi, la zone entre les parois du tube de tige (2) étant configurée sous forme d'évacuation de fluide de rinçage.

8. Résectoscope selon l'une quelconque des revendications précédentes,
**caractérisé en ce que**
l'électrode (11) de l'agencement d'électrode (10) est une électrode bipolaire, de préférence une électrode à boucle ou une électrode de vaporisation plane.

9. Résectoscope selon l'une quelconque des revendications précédentes,
**caractérisé en ce que**
la longueur de course (90) est de 24 mm et/ou la longueur (91) des bras (12) du sous-groupe de fourche (13) est de 30 mm à 28 mm, de préférence de 32 mm à 34 mm.

10. Agencement d'électrode (10) pour utilisation dans un résectoscope (1) ayant une longueur de course de 24 mm, l'agencement d'électrode (10) pouvant être déployé dans la direction longitudinale de la longueur de course (90), la longueur de course (90) désignant la longueur entre les points d'extrémité de la mobilité de l'agencement d'électrode (10), comprenant une électrode (11) qui est agencée à l'extrémité distale des bras (12) d'un sous-groupe de fourche (13) qui sont réunis dans une zone de transition (14) en une tige d'électrode (15), la longueur (91) des bras (12) du sous-groupe de fourche (13) étant supérieure à 1,2 fois la longueur de course (90).
